# EUROPEAN PATENT SPECIFICATION

(11) **EP 1 465 875 B1**
(45) Date of publication and mention of the grant of the patent: **19.12.2007**
(21) Application number: 02795133.4
(22) Date of filing: 10.12.2002
(51) Int. Cl.: C07D 239/47, A61K 31/506, A61P 9/00, C07D 239/56, C07D 239/52, C07D 239/48, C07D 401/14, C07D 403/14

(54) **NOVEL ALKANSULFONAMIDES AS ENDOTHELIN ANTAGONISTS**
NEUE ALKYLSULFONAMIDE ALS ENDOTHELINANTAGONISTEN
ALCANESULFONAMIDES EN TANT QU'ANTAGONISTES DES ENDOTHELINES

(30) Priority: 02.01.2002 WO PCT/EP02/00002
(43) Date of publication of application: 13.10.2004
(73) Proprietor: Actelion Pharmaceuticals Ltd., 4123 Allschwil (CH)
(72) Inventor: BOLLI, Martin, CH-4123 Allschwil (CH); BOSS, Christoph, CH-4123 Allschwil (CH); CLOZEL, Martine, CH-4102 Binningen (CH); FISCHLI, Walter, CH-4123 Allschwil (CH); WELLER, Thomas, CH-4102 Binningen (CH)
(74) Representative: Ruhlmann, Eric
(86) International application number: PCT/EP2002/013970
(87) International publication number: WO 2003/055863

(56) References cited:
- EP-A- 0 743 307
- EP-A- 1 191 026
- WO-A-01/17976
- HARADA, HIRONORI ET AL: "Ethenesulfonamide and ethanesulfonamide derivatives, a novel class of orally active endothelin-A receptor antagonists" BIOORGANIC & MEDICINAL CHEMISTRY (2001), 9(11), 2955-2968 , XP002238534

## Description

### Introduction:

The present invention relates to novel alkanesulfonamides of the formula VI and their use as active ingredients in the preparation of pharmaceutical compositions. The invention also concerns related aspects including processes for the preparation of the compounds, pharmaceutical compositions containing one or more compounds of the formula VI and especially their use as endothelin receptor antagonists.

Endothelins (ET-1, ET-2, and ET-3) are 21-amino acid peptides produced and active in almost all tissues (Yanagisawa M et al.: Nature (1988) 332:411). Endothelins are potent vasoconstrictors and important mediators of cardiac, renal, endocrine and immune functions (McMillen MA et al.: J Am Coll Surg (1995) 180:621). They participate in bronchoconstriction and regulate neurotransmitter release, activation of inflammatory cells, fibrosis, cell proliferation and cell differentiation (Rubanyi GM et al.: Pharmacol Rev (1994) 46:328).

Two endothelin receptors have been cloned and characterized in mammals (ET_{A}, ET_{B}) (Arai H et al.: Nature (1990) 348:730; Sakurai T et al.: Nature (1990) 348:732). The ET_{A} receptor is characterized by higher affinity for ET-1 and ET-2 than for ET-3. It is predominant in vascular smooth muscle cells and mediates vasoconstricting and proliferative responses (Ohlstein EH et al.: Drug Dev Res (1993) 29:108). In contrast, the ET_{B} receptor has equivalent affinity for the three endothelin isopeptides and binds the linear form of endothelin, tetra-ala-endothelin, and sarafotoxin S6C (Ogawa Y et al.: BBRC (1991) 178:248). This receptor is located in the vascular endothelium and smooth muscles, and is also particularly abundant in lung and brain. The ET_{B} receptor from endothelial cells mediates transient vasodilator responses to ET-1 and ET-3 through the release of nitric oxide and/or prostacyclin whereas the ET_{B} receptor from smooth muscle cells exerts vasoconstricting actions (Sumner MJ et al.: Brit J Pharmacol (1992) 107:858). ET_{A} and ET_{B} receptors are highly similar in structure and belong to the superfamily of G-protein coupled receptors.

A pathophysiological role has been suggested for ET-1 in view of its increased plasma and tissue levels in several disease states such as hypertension, pulmonary hypertension, sepsis, atherosclerosis, acute myocardial infarction, congestive heart failure, renal failure, migraine and asthma. As a consequence, endothelin receptor antagonists have been studied extensively as potential therapeutic agents. Endothelin receptor antagonists have demonstrated preclinical and/or clinical efficacy in various diseases such as cerebral vasospasm following subarachnoid hemorrhage, heart failure, pulmonary and systemic hypertension, neurogenic inflammation, renal failure and myocardial infarction.

Today, only one endothelin receptor antagonist (bosentan, Tracleer^{™}) is marketed and several are in clinical trials. However, some of these molecules possess a number of weaknesses such as complex synthesis, low solubility, high molecular weight, poor pharmacokinetics, or safety problems (e.g. liver enzyme increases). Furthermore, the contribution of differing ET_{A} / ET_{B} receptor blockade to the clinical outcome is not known. Thus, tailoring of the physicochemical and pharmacokinetic properties and the selectivity profile of each antagonist for a given clinical indication is mandatory. So far, no endothelin receptor antagonists with a pyrimidine core structure containing an n-alkanesulfonamide unit attached to the 4-position of the core pyrimidine have been reported [2, 3, 5, 6, 8].

WO 01/17976 A describes endothelin receptor antagonists with a pyrimidine core structure containing an aryl-, aralkyl-, arylalkenyl-, heteroaryl- or heteroarylalkyl-sulfonamide unit attached to the 4-position of the core pyrimidine,

An article by Harada et al. ("Ethenesulfonamide and ethanesulfonamide derivatives, a novel class of orally active endothelin-A receptor antagonists", Bioorg. Med. Chem. (2001), 9, 2955-2968) discusses the endothelin receptor antagonist activity of compounds with a pyrimidine core structure containing an arylalkenyl-, heteroarylalkenyl- or aralkyl-sulfonamide unit attached to the 4-position of the core pyrimidine.

EP-A-0 743 307 relates notably to endothelin receptor antagonists with a pyrimidine core structure containing a cycloalkylalkyl-, heterocyclylalkyl, aralkyl- or heteroarylalkyl-sulfonamide unit attached to the 4-position of the core pyrimidine and a pyridazine attached to the 2-position of the core pyrimidine.

We have discovered a new class of substituted pyrimidines of the formula VI below and found that they allow the specific tailoring described above.

In addition, compounds exhibiting mixed as well as ET_{A}-selective binding profiles have been identified.

The inhibitory activity of the compounds of formula VI on endothelin receptors can be demonstrated using the test procedures described hereinafter:

*For the evaluation of the potency and efficacy of the compounds of the formula VI the following tests were used:*

### 1) Inhibition of endothelin binding to membranes from CHO cells carrying human ET receptors:

For competition binding studies, membranes of CHO cells expressing human recombinant ET_{A} or ET_{B} receptors were used. Microsomal membranes from recombinant CHO cells were prepared and the binding assay was carried out as previously described (Breu V., et al, FEBS Lett 1993; 334:210).

The assay was performed in 200 uL 50 mM Tris/HCl buffer, pH 7.4, including 25 mM MnCl₂, 1 mM EDTA and 0.5% (w/v) BSA in polypropylene microtiter plates. Membranes containing 0.5 ug protein were incubated for 2 h at 20°C with 8 pM [²⁵I]ET-1 (4000 cpm) and increasing concentrations of unlabelled antagonists. Maximum and minimum binding were estimated in samples without and with 100 nM ET-1, respectively. After 2 h, the membranes were filtered on filterplates containing GF/C filters (Unifilterplates from Canberra Packard S.A. Zürich, Switzerland). To each well, 50 uL of scintillation cocktail was added (MicroScint 20, Canberra Packard S.A. Zürich, Switzerland) and the filter plates counted in a microplate counter (TopCount, Canberra Packard S.A. Zürich, Switzerland).

All the test compounds were dissolved, diluted and added in DMSO. The assay was run in the presence of 2.5% DMSO which was found not to interfere significantly with the binding. IC₅₀was calculated as the concentration of antagonist inhibiting 50 % of the specific binding of ET-1. For reference compounds, the following IC₅₀ values were found: *ET_{A} cells:* 0.075 nM (n=8) for ET-1 and 118 nM (n=8) for ET-3; *ET_{B} cells:* 0.067 nM (n=8) for ET-1 and 0.092 nM (n=3) for ET-3.

The IC₅₀ values obtained with compounds of formula VI are given in Table 1.

**Table 1:**

| Compound of Example | IC₅₀[nM] | |
|---|---|---|
| | ET_{A} | ET_{B} |
| Example 1 | 3.96 | >1000 |
| Example 2 | 38.2 | >1000 |
| Example 3 | 6.34 | >1000 |
| Example 4 | 17.1 | >1000 |
| Example 6 | 0.66 | 838 |
| Example 7 | 6.8 | > 1000 |
| Example 8 | 1.5 | > 1000 |
| Example 9 | 13 | > 1000 |
| Example 10 | 0.46 | > 1000 |
| Example 11 | 3.6. | > 1000 |
| Example 12 | 0.63 | > 1000 |
| Example 13 | 3.5 | > 1000 |
| Example 14 | 0.54 | > 1000 |
| Example 15 | 1.27 | > 1000 |

### 2) Inhibition of endothelin-induced contractions on isolated rat aortic rings (ET_{A} receptors) and rat tracheal rings (ET_{B} receptors):

The functional inhibitory potency of the endothelin antagonists was assessed by their inhibition of the contraction induced by endothelin-1 on rat aortic rings (ET_{A} receptors) and of the contraction induced by sarafotoxin S6c on rat tracheal rings (ET_{B} receptors). Adult Wistar rats were anesthetized and exsanguinated. The thoracic aorta or trachea were excised, dissected and cut into rings of 3-5 mm lenght. The endothelium/epithelium was removed by gentle rubbing of the intimal surface. Each ring was suspended in a 10 ml isolated organ bath filled with Krebs-Henseleit solution (in mM; NaCl 115, KCl 4.7, MgSO₄ 1.2, KH₂PO₄ 1.5, NaHCO₃ 25, CaCl₂ 2.5, glucose 10) kept at 37°C and gassed with 95% O₂ and 5% CO₂. The rings were connected to force transducers and isometric tension was recorded (EMKA Technologies SA, Paris, France). The rings were stretched to a resting tension of 3 g (aorta) or 2 g (trachea). Cumulative doses of ET-1 (aorta) or sarafotoxin S6c (trachea) were added after a 10 min incubation with the test compound or its vehicle. The functional inhibitory potency of the test compound was assessed by calculating the concentration ratio, i.e. the shift to the right of the EC₅₀ induced by different concentrations of test compound, EC₅₀ is the concentration of endothelin needed to get a half-maximal contraction, pA₂ is the negative logarithm of the antagonist concentration which induces a two-fold shift in the EC₅₀ value.

Because of their ability to inhibit the endothelin binding, the described compounds can be used for treatment of diseases, which are associated with an increase in vasoconstriction, proliferation or inflammation due to endothelin. Examples of such diseases are hypertension, pulmonary hypertension, coronary diseases, cardiac insufficiency, renal and myocardial ischemia, renal failure, cerebral ischemia, dementia, migraine, subarachnoidal hemorrhage, Raynaud's syndrome and portal hypertension. They can also be used in the treatment or prevention of atherosclerosis, restenosis after balloon or stent angioplasty, inflammation, stomach and duodenal ulcer, cancer, prostatic hypertrophy, erectile dysfunction, hearing loss, amaurosis, chronic bronchitis, asthma, gram negative septicemia, shock, sickle cell anemia, glomerulonephritis, renal colic, glaucoma, therapy and prophylaxis of diabetic complications, complications of vascular or cardiac surgery or after organ transplantation, complications of cyclosporin treatment, pain, hyperlipidemia as well as other diseases, presently known to be related to endothelin.

The compounds can be administered orally, rectally, parenterally, e.g. by intravenous, intramuscular, subcutaneous, intrathecal or transdermal administration or sublingually or as ophthalmic preparation or administered as aerosol. Examples of applications are capsules, tablets, orally administered suspensions or solutions, suppositories, injections, eye-drops, ointments or aerosols/nebulizers.

Preferred applications are intravenous, intra-muscular, or oral administrations as well as eye drops. The dosage used depends upon the type of the specific active ingredient, the age and the requirements of the patient and the kind of application. Generally, dosages of 0.1 - 50 mg / kg body weight per day are considered. The preparations with compounds can contain inert or as well pharmacodynamically active excipients. Tablets or granules, for example, could contain a number of binding agents, filling excipients, carrier substances or diluents.

### Description of the Invention:

The invention consists of the compounds described in formula **VI** and their use as endothelin receptor antagonists and especially their use as medicaments for the treatment and prevention of diseases related to the endothelin system: 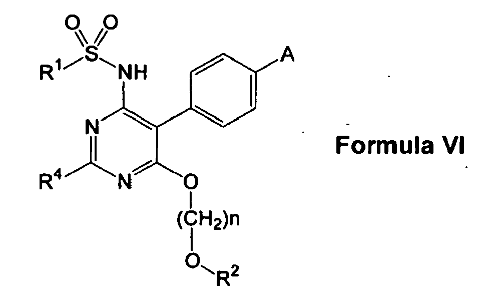 wherein
- **R¹**: represents ethyl; propyl; butyl;
- **R²**: represents aryl; heteroaryl;
- **R⁴**: represents hydrogen or heteroaryl;
- **n**: represents the integers 2; 3;
and optically pure enantiomers or diastereomers, mixtures of enantiomers or diastereomers, diastereomeric racemates, mixtures of diastereomeric racemates and the meso-forms and pharmaceutically acceptable salts thereof.

In the definitions of the formula VI - if not otherwise stated - the expression "heterocyclyl" means saturated or unsaturated (but not aromatic), four, five-, six- or seven-membered rings containing one or two nitrogen, oxygen or sulfur atoms which may be the same or different and which rings may be adequately substituted with lower alkyl, lower alkoxy; examples of heterocyclyl are piperidinyl, morpholinyl, thiomorpholinyl, piperazinyl, tetrahydropyranyl, dihydropyranyl, 1,4-dioxanyl, pyrrolidinyl, tetrahydrofuranyl, dihydropyrrolyl, dihydroimidazolyl, dihydropyrazolyl, pyrazolidinyl and substituted derivatives of such rings with substituents as outlined above. The expression "heteroaryl" means six-membered aromatic rings containing one to four nitrogen atoms, benzofused six-membered aromatic rings containing one to three nitrogen atoms, five-membered aromatic rings containing one oxygen or one nitrogen or one sulfur atom, benzofused five-membered aromatic rings containing one oxygen or one nitrogen or one sulfur atom, five membered aromatic rings containing an oxygen and nitrogen atom and benzo fused derivatives thereof, five-membered aromatic rings containing a sulfur and a nitrogen atom and benzo fused derivatives thereof, five-membered aromatic rings containing two nitrogen atoms and benzo fused derivatives thereof, five membered aromatic rings containing three nitrogen atoms and benzo fused derivatives thereof or the tetrazolyl ring; e.g. furanyl, thienyl, pyrrolyl, pyridinyl, pyrimidinyl, indolyl, quinolinyl, isoquinolinyl, imidazolyl, triazinyl, thiazinyl, thiazolyl, isothiazolyl, pyridazinyl, oxazolyl, isoxazolyl, 5-oxo-1,2,4-oxadiazolyl, 5-oxo-1,2,4-thiadiazolyl, 5-thioxo-1,2,4-oxadiazolyl, 2-oxo-1,2,3,5-oxathiadiazolyl, whereby such rings may be substituted with alkyl of 1 to 7 carbon atoms, alkenyl of 2 to 7 carbon atoms, amino, amino-alkyl of 1 to 7 carbon atoms, halogen, hydroxy, alkoxy of 1 to 7 carbon atoms, trifluoromethoxy, trifluoromethyl, carboxyl, carboxamidyl, thioamidyl, amidinyl, alkoxy-carbonyl wherein the alkoxy is an alkoxy of 1 to 7 carbon atoms, cyano, hydroxy- alkyl of 1 to 7 carbon atoms, alkoxy-alkyl wherein the alkoxy is an alkoxy of 1 to 7 carbon atoms and the alkyl is an alkyl of 1 to 7 carbon atoms or another heteroaryl- or heterocyclyl-ring. The expression "aryl" represents unsubstituted as well as mono-, di- or tri-substituted phenyl or naphthyl rings which may be substituted with aryl, halogen, hydroxy, alkyl of 1 to 7 carbon atoms, alkenyl of 2 to 7 carbon atoms, alkynyl of 2 to 7 carbon atoms, alkoxy of 1 to 7 carbon atoms, alkenyloxy of 2 to 7 carbon atoms, lalkynyl-alkoxy wherein the alkynyl is an alkynyl of 2 to 7 carbon atoms and the alkoxy is an alkoxy of 1 to 7 carbon atoms, alkenylen of 1 to 7 carbon atoms, allyloxy, vinyloxy or propenyloxy, methylendioxy or ethylendioxy forming with the phenyl ring a five- or six-membered ring, hydroxy-alkyl of 1 to 7 carbon atoms, hydroxy-alkenyl of 2 to 7 carbon atoms, hydroxy-alkyl-alkynyl wherein the alkyl is an alkyl of 1 to 7 carbon atoms and the alkynyl is an alkynyl of 2 to 7 carbon atoms, alkoxy-alkyl wherein the alkoxy is an alkoxy of 1 to 7 carbon atoms and the alkyl is an alkyl of 1 to 7 carbon atoms, alkoxy-alkoxy wherein each alkoxy is an alkoxy of 1 to 7 carbon atoms, trifluoromethyl, trifluoromethoxy, cycloalkyl of 3 to 7 carbon atoms optionally substituted by groups selected from alkyl of 1 to 7 carbon atoms, hydroxy-alkyl of 1 to 7 carbon atoms, amino-alkyl of 1 to 7 carbon atoms and alkoxy-alkyl wherein the alkoxy is an alkoxy of 1 to 7 carbon atoms and the alkyl is an alkyl of 1 to 7 carbon atoms, hydroxy-cycloalkyl, heterocyclyl, heteroaryl.

The expression pharmaceutically acceptable salts encompasses either salts with inorganic acids or organic acids like hydrohalogenic acids, e.g. hydrochloric or hydrobromic acid; sulfuric acid, phosphoric acid, nitric acid, citric acid, formic acid, acetic acid, maleic acid, tartaric acid, methylsulfonic acid, p- toluolsulfonic acid and the like or in case the compound of formula VI is acidic in nature with an inorganic base like an alkali or earth alkali base, e.g. sodium hydroxide, potassium hydroxide, calcium hydroxide and the like.

The compounds of the formula VI might have one or more asymmetric carbon atoms and may be prepared in form of optically pure enantiomers or diastereomers, mixtures of enantiomers or diastereomers, diastereomeric racemates, mixtures of diastereomeric racemates and also in the meso-form. The present invention encompasses all these forms. Mixtures may be separated in a manner known per se, i.e. by column chromatography, thin layer chromatography, HPLC or crystallization.

Because of their ability to inhibit the endothelin binding, the described compounds of the formula VI and their pharmaceutically acceptable salts may be used for treatment of diseases which are associated with an increase in vasoconstriction, proliferation or inflammation due to endothelin. Examples of such diseases are hypertension, coronary diseases, cardiac insufficiency, renal and myocardial ischemia, renal failure, cerebral ischemia, dementia, migraine, subarachnoidal hemorrhage, Raynaud's syndrome, portal hypertension and pulmonary hypertension. They can also be used for the treatment or prevention of atherosclerosis, restenosis after balloon or stent angioplasty, inflammation, stomach and duodenal ulcer, cancer, prostatic hypertrophy, erectile dysfunction, hearing loss, amaurosis, chronic bronchitis, asthma, gram negative septicemia, shock, sickle cell anemia, glomerulonephritis, renal colic, glaucoma, therapy and prophylaxis of diabetic complications, complications of vascular or cardiac surgery or after organ transplantation, complications of cyclosporin treatment, pain, hyperlipidemia as well as other diseases presently known to be related to endothelin.

These compositions may be administered in enteral or oral form e.g. as tablets, dragees, gelatine capsules, emulsions, solutions or suspensions, in nasal form like sprays or rectally in form of suppositories. These compounds may also be administered intramuscularly, parenterally or intraveneously, e.g. in form of injectable solutions.

These pharmaceutical compositions may contain the compounds of formula VI as well as their pharmaceutically acceptable salts in combination with inorganic and/or organic excipients which are usual in the pharmaceutical industry like lactose, maize or derivatives thereof, talcum, stearinic acid or salts of these materials.

For gelatine capsules vegetable oils, waxes, fats, liquid or half-liquid polyols may be used. For the preparation of solutions and sirups e.g. water, polyols, saccharose, glucose can be used. Injectables can be prepared by using e.g. water, polyols, alcohols, glycerin, vegetable oils, lecithin or liposomes. Suppositories may be prepared by using natural or hydrogenated oils, waxes, fatty acids (fats), liquid or half-liquid polyols.

The compositions may contain in addition preservatives, stability improving substances, viscosity improving or regulating substances, solubility improving substances, sweeteners, dyes, taste improving compounds, salts to change the osmotic pressure, buffer or anti-oxidants.

The compounds of formula VI may also be used in combination with one or more other therapeutically useful substances e.g. α- and β-blockers like phentolamine, phenoxybenzamine, atenolol, propranolol, timolol, metoprolol, carteolol and the like; vasodilators like hydralazine, minoxidil, diazoxide or flosequinan; calcium-antagonists like diltiazem, nicardipine, nimodipine, verapamil or nifedipine; ACE-inhibitors like cilazapril, captopril, enalapril, lisinopril and the like; potassium activators like pinacidil; angiotensin II receptor antagonists like losartan, valsartan, irbesartan and the like; diuretics like hydrochlorothiazide, chlorothiazide, acetolamide, bumetanide, furosemide, metolazone or chlortalidone; sympatholitics like methyldopa, clonidine, guanabenz or reserpine and other therapeutics which serve to treat high blood pressure or any cardiac disorders.

The dosage may vary within wide limits but should be adapted to the specific situation. In general the dosage given daily in oral form should be between about 3 mg and about 3 g, preferably between about 10 mg and about 1 g, especially preferred between 5 mg and 300 mg, per adult with a body weight of about 70 kg. The dosage should be administered preferably in 1 to 3 doses per day which are of equal weight. As usual children should receive lower doses which are adapted to body weight and age.

Preferred compounds of formula VI are the compounds of **formula VII**: 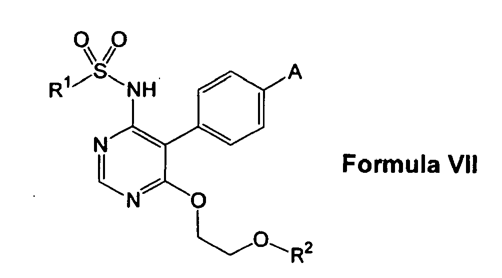 wherein
- **R¹**: represents ethyl; propyl; butyl;
- **R²**: represents heteroaryl;
- **A**: represents methyl; chlorine; bromine;
and optically pure enantiomers or diastereomers, mixtures of enantiomers or diastereomers, diastereomeric racemates, mixtures of diastereomeric racemates and the meso-forms and pharmaceutically acceptable salts thereof.

Preferred compounds are:
Ethanesulfonic acid {6-[2-(5-bromo-pyrimidin-2-yloxy)-ethoxy]-5-p-tolylpyrimidin-4-yl}-amide;
n-Propanesulfonic acid {6-[2-(5-bromo-pyrimidin-2-yloxy)-ethoxy)-5-p-tolylpyrimidin-4-yl}-amide;
Ethanesulfonic acid [6-[2-(5-bromo-pyrimidin-2-yloxy)-ethoxy]-5-(4-chlorophenyl)-pyrimidin-4-yl]-amide;
n-Propanesulfonic acid [6-[2-(5-bromo-pyrimidin-2-yloxy)-ethoxy]-5-(4-chlorophenyl)-pyrimidin-4-yl]-amide;
Ethanesulfonic acid {5-(4-bromo-phenyl)-6-[2-(5-bromo-pyrimidin-2-yloxy)-ethoxy]-pyrimidin-4-yl}-amide;
n-Propanesulfonic acid {5-(4-bromo-phenyl)-6-[2-(5-bromo-pyrimidin-2-yloxy)-ethoxy]-pyrimidin-4-yl}-amide;
Ethanesulfonic acid [6-[2-(5-bromo-pyrimidin-2-yloxy)-ethoxy]-5-(2-methoxyphenoxy)-[2,2']bipyrimidinyl-4-yl]-amide;
N-[6-[2-(5-Bromo-pyrimidin-2-yloxy)-ethoxy]-5-p-tolyl-pyrimidin-4-yl]-methanesulfonamide;

*Especially preferred compounds are:*
N-[5-(4-Bromo-phenyl)-6-[2-(5-bromo-pyrimidin-2-yloxy)-ethoxy]-pyrimidin-4-yl]-methanesulfonamide;
Ethanesulfonic acid [5-(4-bromo-phenyl)-6-[2-(5-bromo-pyrimidin-2-yloxy)-ethoxy]-pyrimidin-4-yl]-amide;
Propane-1-sulfonic acid [5-(4-bromo-phenyl)-6-[2-(5-bromo-pyrimidin-2-yloxy)-ethoxy]-pyrimidin-4-yl]-amide ;

Compounds of the formula VI of the present invention can be prepared according to the general sequence of reactions retro-synthetically outlined below. For simplicity and clarity reasons sometimes only parts of the synthetic possibilities which lead to compounds of formula VI are described. The literature references given in brackets [ ] are set forth at the end of this paragraph.

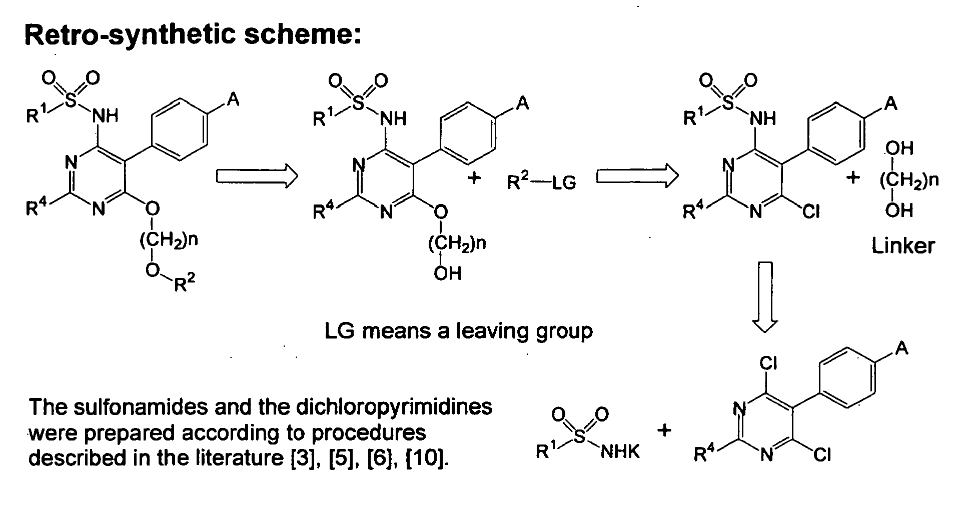

As a general different synthetic possibility, in certain cases the side-chain should first be prepared (especially when R₂ represents aryl) and only then the whole side chain should be attached to the pyrimidine core.

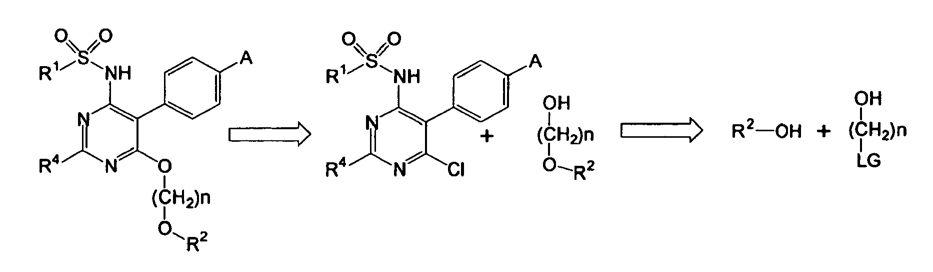

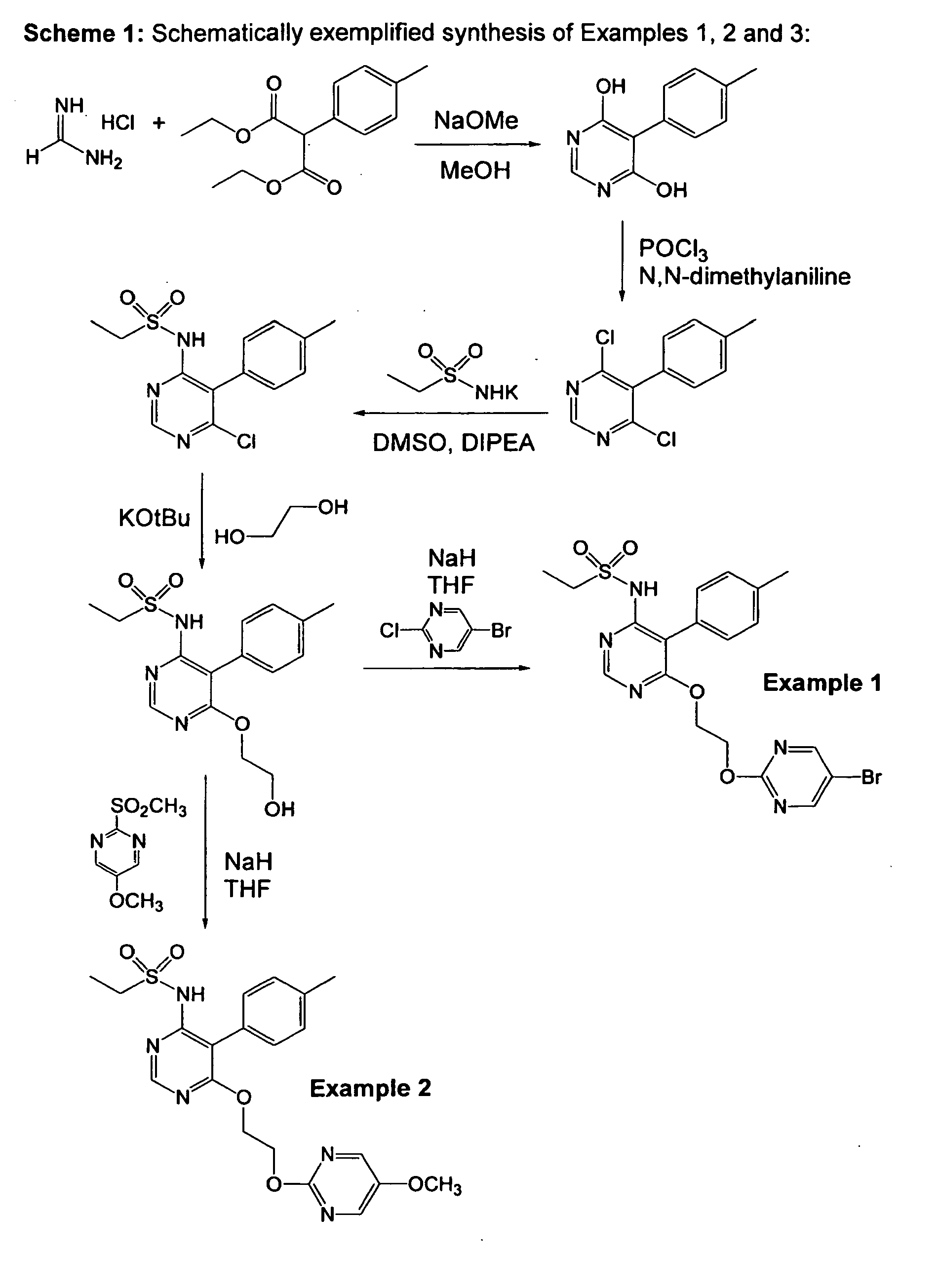

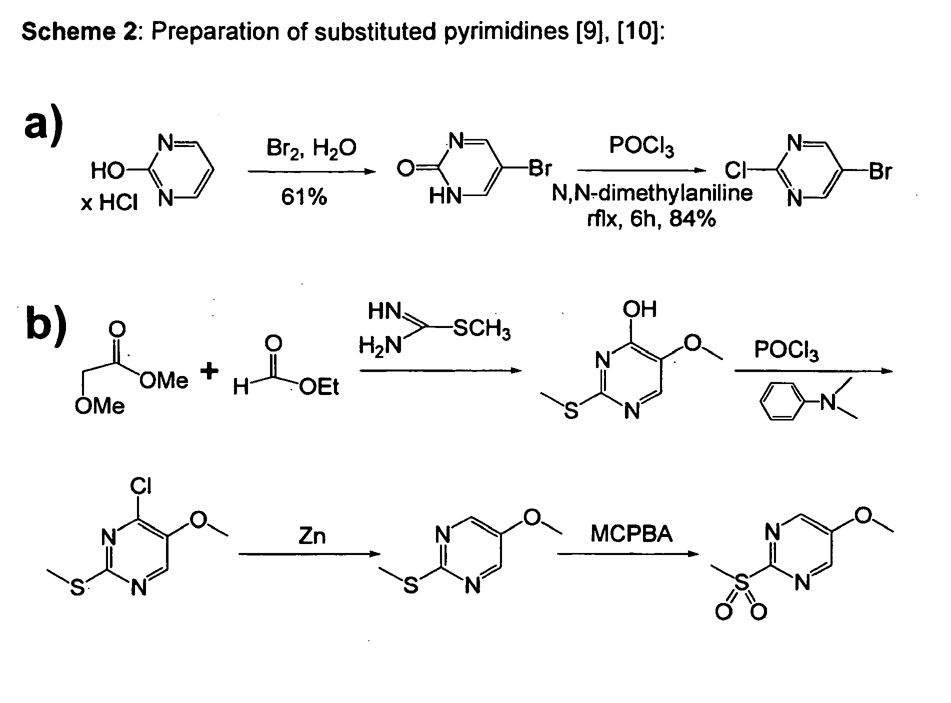

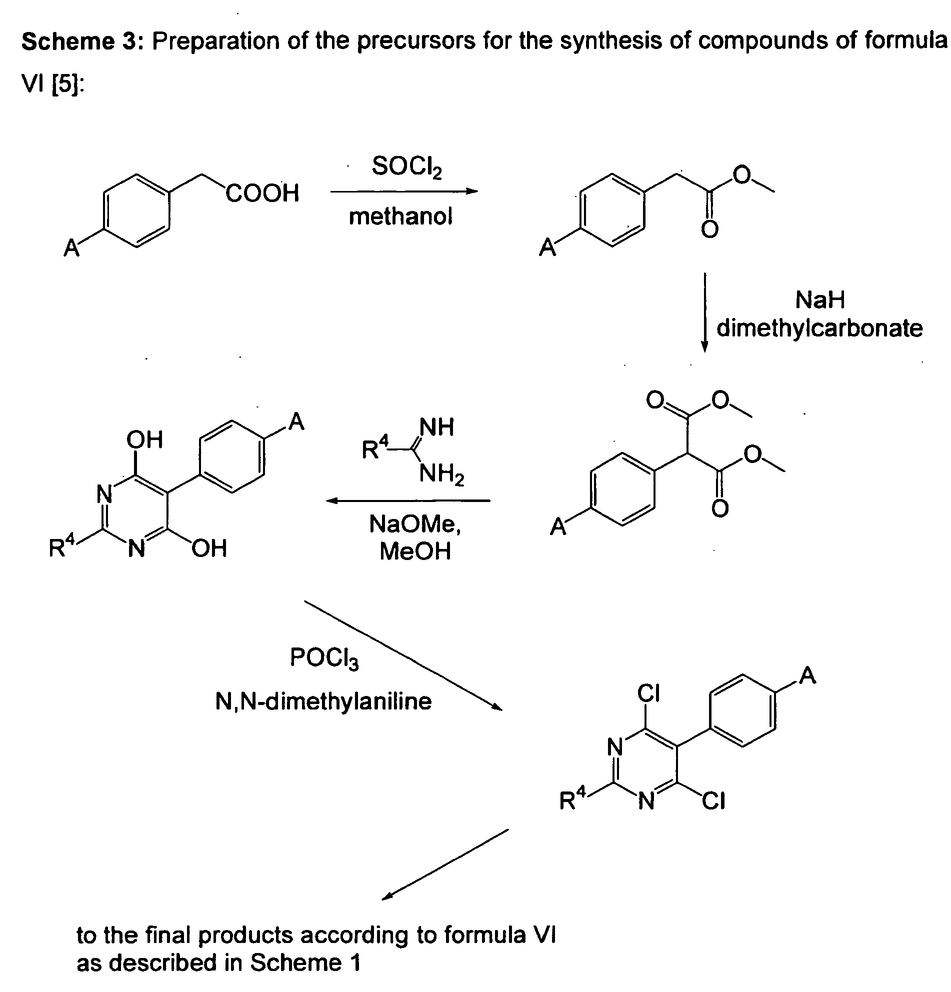

In Scheme 3 the symbols A and R⁴ represent the same as defined in formula VI above.

### References:

[1] W. G6hring, J. Schildknecht, M. Federspiel; Chimia, 1996, 50, 538 - 543.
[2] W. Neidhart, V. Breu, D. Bur, K. Burri, M. Clozel, G. Hirth, M. Müller, H. P. Wessel, H. Ramuz; Chimia, 1996, 50, 519 - 524 and references cited there.
[3] W. Neidhart, V. Breu, K. Burri, M. Clozel, G. Hirth, U. Klinkhammer, T. Giller, H. Ramuz; Bioorg. Med. Chem. Lett., 1997, 7, 2223 - 2228. R. A. Nugent, S. T. Schlachter, M. J. Murphy, G. J. Cleek, T. J. Poel, D. G. Whishka, D. R. Graber, Y. Yagi, B. J. Keiser, R. A. Olmsted, L. A: Kopta, S. M. Swaney, S. M. Poppe, J. Morris, W. G. Tarpley, R. C. Thomas; J. Med. Chem., 1998, 41, 3793 - 3803.
[4] J. March; Advanced Organic Chemistry, 4th Ed., 1994, p. 499 and references cited there.
[5] EP 0 743 307 A1; EP 0 658 548 B1; EP 0 959 072 A1 (Tanabe Seiyaku)
[6] EP 0 633 259 B1; EP 0 526 708 A1; WO 96/19459 (F. Hoffmann-LaRoche)
[7] for the Synthesis of 5-membered heterocycles see: Y. Kohara et al; J. Med. Chem., 1996, 39, 5228 - 5235 and references cited there.
[8] EP 0 882 719 A1 (Yamanouchi Pharmaceutical Co., Ltd)
[9] D. G. Crosby, R. V. Berthold; J. Org. Chem., 1960; 25; 1916.
[10] US-4,233,294, 1980, (Bayer AG);
[11] WO 01/17976; WO 01/46156; WO 01/81335; WO 01/81338; WO 02/24665; WO 02/208200 (Actelion Pharmaceuticals Ltd);

### Examples

The following examples illustrate the invention. All temperatures are stated in °C.

### List of Abbreviations:

- CyHex: cyclohexane
- DBU: 1,8-diazabicyclo[5.4.0]undec-7-en(1,5-5)
- DCM: dichloromethane
- DIPEA: diisopropylethylamine
- DMAP: 4-dimethylaminopyridine
- DMF: dimethylformamide
- DMSO: dimethylsulfoxide
- EtOAc: ethyl acetate
- Hex: hexane
- HV: high vacuum conditions
- MCPBA: m-chloroperbenzoic acid
- min: minutes
- rflx: reflux
- rt: room temperature
- THF: tetrahydrofuran
- t_{R}: retention time

The following compounds were prepared according to the procedure described above and shown in *Schemes 1 to* 3*.* All compounds were characterized by 1H-NMR (300MHz) and occasionally by 13C-NMR (75MHz) (Varian Oxford, 300MHz; chemical shifts are given in ppm relative to the solvent used; multiplicities: s = singlet, d = doublet, t = triplet; m = multiplet), by LC-MS (Waters Micromass; ZMD-platform with ESI-probe with Alliance 2790 HT; Column: 2x30mm, Gromsil ODS4, 3µm, 120A; Gradient: 0 - 100% acetonitril in water, 6 min, with 0.05% formic acid, flow: 0.45ml/min; t_{R} is given in min.) or by Finnigan Navigator (LC-MS¹) with HP 1100 Binary Pump and DAD, column: 4.6x50 mm, Develosil RP Aqueous, 5 µm, 120A, gradient: 5-95% acetonitrile in water, 1 min, with 0.04% trifluoroacetic acid, flow: 4.5 ml/min) by TLC (TLC-plates from Merck, Silica gel 60 F₂₅₄) and occasionally by melting point.

### Example 1:

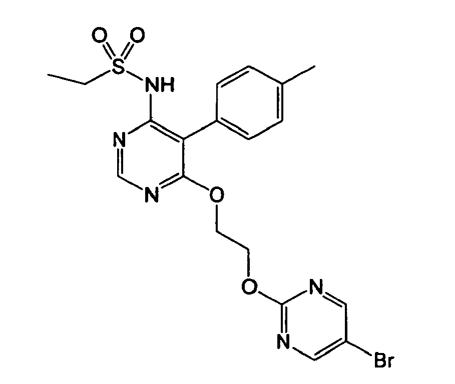

a) At 0°C a solution of diethyl 2-(p-tolyl)-malonate (14.2 g) in methanol (50 ml) was slowly added to a solution of sodium methylate (9.4 g) in methanol (300 ml). Upon completion of the addition the reaction mixture was allowed to warm up and formamidine hydrochloride (5.4 g) was added. The mixture was stirred at rt for 16 h. The solvent was removed under reduced pressure and the remaining residue was treated with 2 N hydrochloric acid (150 ml). The suspension was stirred for 0.5 h. At 0-5°C, the pH was carefully adjusted to 4 using 10 N sodium hydroxide solution. The precipitate was collected, washed with cold water, isopropanol, and diethyl ether and dried under high vacuum at 65°C to give 4,6-dihydroxy-5-(p-tolyl)-pyrimidine (11.2 g) (or a tautomer) as a white powder.
b) At rt N,N-dimethylaniline (10 ml) was added to a mixture of 4,6-dihydroxy-5-(p-tolyl)-pyrimidine (5.1 g) and POCl₃ (75 ml). The reaction mixture was stirred at 70°C for 16 h. The excess of POCl₃ was distilled off and the remaining oil was treated with an ice:water mixture and extracted three times with diethyl ether. The combined organic extracts were washed with 1 N aqueous hydrochloric acid followed by brine, dried over MgSO₄ and evaporated. The remaining brown oil was crystallised from isopropanol. The pale yellow crystals were collected, washed with cold isopropanol and dried under high vacuum to furnish 4,6-dichloro-5-(p-tolyl)-pyrimidine (4.1 g).
c) Ethanesulfonyl chloride (24 g) was dissolved in THF (30 ml) and cooled to 0°C. Then ammonium hydroxide solution (25%, 40 ml) was added via addition funnel followed by stirring at rt for 1 h. The THF was removed under reduced pressure and the remaining solution was extracted with ethyl acetate. The combined organic layers were dried over magnesium sulfate and concentrated in vacuo to give ethanesulfonamide (7.2 g) as an oil which was dissolved in MeOH (100 ml) followed by the addition of potassium tert.-butoxide (7.4 g) and stirring for 30 min. The solvent was evaporated and the residue was washed with diethyl ether and dried at HV to give ethanesulfonamide potassium salt (9.7 g) as a white, hygroscopic powder.
d) 4,6-dichloro-5-(p-tolyl)-pyrimidine (717 mg) was dissolved in DMSO (5 ml) and ethanesulfonamide potassium salt (927 mg) was added and stirring continued for 14 h at rt. The solution was poured onto ice/water and acidified by 2 N HCl to pH 3-4. The precipitate was filtered off and washed with water and diethylether to give ethanesulfonic acid (6-chloro-5-p-tolyl-pyrimidin-4-yl)-amide (370 mg) as a white powder. LC-MS: t_{R}: 4.09, [M+H)⁺: 312.10.
e) Ethanesulfonic acid (6-chloro-5-p-tolyl-pyrimidin-4-yl)-amide (363 mg) was added to a solution of potassium tert.-butoxide (427 mg) in ethylene glycol (7 ml) and stirred at 100°C for 7 days. The reaction mixture was then poured onto ice/water and extracted with ethyl acetate. The crude product was purified by chromatography over silicagel with DCM / MeOH = 9 / 1 to give ethanesulfonic acid [6-(2-hydroxyethoxy)-5-p-tolyl-pyrimidin-4-yl]-amide (310 mg) as a white powder. LC-MS: t_{R}: 3.47, [M+H]⁺: 338.13.
f) Ethanesulfonic acid [6-(2-hydroxy-ethoxy)-5-p-tolyl-pyrimidin-4-yl]-amide (135 mg) was dissolved in THF (15 ml) and sodium hydride (80 mg) was added followed by stirring for 15 min at 50°C. Then 2-chloro-5-bromo-pyrimidine (162 mg) was added and stirring was continued for 8 h at 70 C. The reaction mixture was poured onto ice water, acidified with solid citric acid and extracted with ethylacetate. The combined organic extracts were dried over magnesium sulfate and the solvent was evaporated. The crude material was purified by plate chromatography with ethyl acetate / hexane = 1 / 2 to give ethanesulfonic acid {6-[2-(5-bromo-pyrimidin-2-yloxy)-ethoxy]-5-p-tolyl-pyrimidin-4-yl}-amide (68 mg) as a white powder. LC-MS: t_{R}: 4.64, [M+H]⁺: 496.19.

### Example 2:

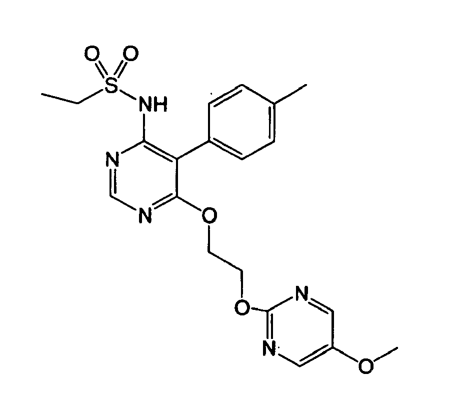

According to the procedure described in Example 1f) ethanesulfonic acid {6-[2-(5-methoxy-pyrimidin-2-yloxy)-ethoxy]-5-p-tolyl-pyrimidin-4-yl}-amide (65 mg) was prepared by reaction of ethanesulfonic acid [6-(2-hydroxy-ethoxy)-5-p-tolyl= pyrimidin-4-yl]-amide (84 mg) with 2-sulfono-5-methoxy-pyrimidine (103 mg). LC-MS: t_{R}: 4.25, [M+H]⁺: 446.35.

### Example 3:

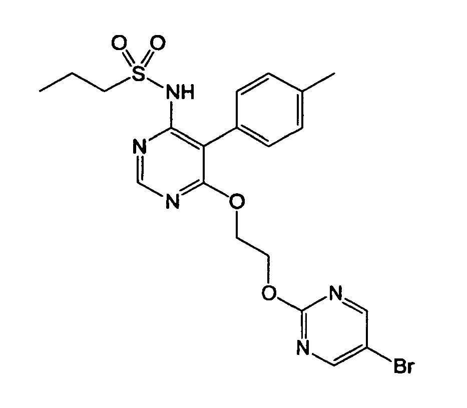

a) n-Propane sulfonyl chloride (20.7 g) was dissolved in THF (40 ml) and cooled to 0°C. Then ammonium hydroxide solution (25%, 40 ml) was added via addition funnel followed by stirring at rt for 1 h. The THF was removed under reduced pressure and the remaining solution was extracted with ethyl acetate. The combined organic extracts were dried over magnesium sulfate and concentrated in vacuo to give n-propane sulfonamide (10.99 g) as an oil which was dissolved in MeOH (100 ml) followed by the addition of potassium tert.-butoxide (10.6 g) and stirring for 30 min. The solvent was evaporated and the residue was triturated with diethyl ether. The white solid was isolated by filtration and dried at HV to give n-propanesulfonamide potassium salt (13.4 g) as a white, hygroscopic powder.
b) To a solution of 4,6-dichloro-5-(p-tolyl)-pyrimidine (Example 1b; 717 mg) in DMSO (5 ml) and n-propanesulfonamide potassium salt (1016 mg) was added. Stirring was continued for 14 h at rt. The solution was poured onto ice/water and acidified by 2 N HCl to pH 3-4. The precipitate was filtered off and washed with water and diethylether to give n-propanesulfonic acid (6-chloro-5-p-tolyl-pyrimidin-4-yl)-amide (765 mg) as a white powder. LC-MS: t_{R}: 4.44, [M+H]⁺: 326.13.
c) n-Propanesulfonic acid (6-chloro-5-p-tolyl-pyrimidin-4-yl)-amide (489 mg) was added to a solution of potassium tert.-butoxide (900 mg) in ethylene glycol (10 ml). The solution was stirred at 100°C for 7 days. The reaction mixture was then poured onto ice/water and extracted with ethyl acetate. The crude product was purified by chromatography over silicagel with DCM / MeOH = 9 / 1 to give n-propanesulfonic acid [6-(2-hydroxy-ethoxy)-5-p-tolyl-pyrimidin-4-yl]-amide (390 mg) as a white powder. LC-MS: t_{R}: 3.76, [M+H]⁺: 352.13.
d) n-Propanesulfonic acid [6-(2-hydroxy-ethoxy)-5-p-tolyl-pyrimidin-4-yl]-amide (115 mg) was dissolved in THF (15 ml). Sodium hydride (60 mg) was added followed by stirring for 15 min at 50°C. Then 2-chloro-5-bromo-pyrimidine (135 mg) was added and stirring was continued for 8h at 75°C. The reaction mixture was poured onto ice water, acidified with solid citric acid and extracted with ethyl acetate. The combined organic extracts were dried over magnesium sulfate, filtered and the solvent was evaporated. The crude material was purified by plate chromatography with diethyl ether to give n-propanesulfonic acid {6-[2-(5-bromo-pyrimidin-2-yloxy)-ethoxy]-5-p-tolyl-pyrimidin-4-yl}-amide (65 mg) as a white powder. LC-MS: t_{R}: 4.91, [M+H]⁺: 510.13.

### Example 4:

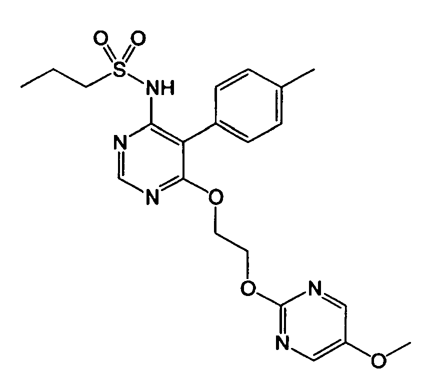

n-Propanesulfonic acid [6-(2-hydroxy-ethoxy)-5-p-tolyl-pyrimidin-4-yl]-amide (115.5 mg) was dissolved in THF (10 ml) and sodium hydride (60 mg) was added followed by stirring for 15 min at 50°C. Then 2-methanesulfonyl-5-methoxy-pyrimidine (138 mg) was added and stirring was continued for 8 h at 75°C. The reaction mixture was poured onto ice water, acidified with solid citric acid and extracted with ethyl acetate. The combined organic layers were dried over magnesium sulfate and the solvent was evaporated. The crude material was purified by plate chromatography with diethyl ether to give propane-1-sulfonic acid {6-[2-(5-methoxy-pyrimidin-2-yloxy)-ethoxy]-5-p-tolyl-pyrimidin-4-yl}-amide (61 mg) as a white powder. LC-MS: t_{R}: 4.51, [M+H]⁺: 460.27.

According to the procedures described in the Examples 1 to 4 and in the literature [5], [6], [7], [8] and [11] the compounds depicted in the following table of Example 5 can be prepared.

### Example 5:

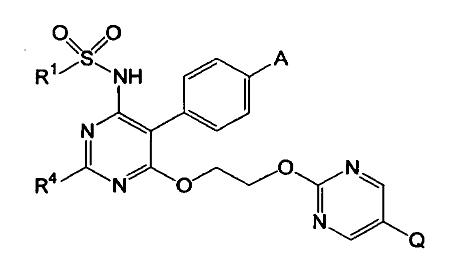

| **R¹:** | **R⁴:** | **A** | **Q** |
|---|---|---|---|
| 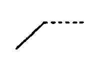 | 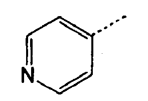 | H---- | Br----- |
| | | H₃C ---- | Cl----- |
| 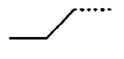 | 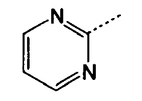 | Cl----- | H₃C ---- |
| 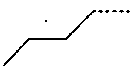 | 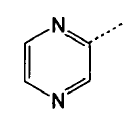 | Br----- | H₃CO ---- |
| | | | H---- |

In the Examples 6 to 16 the retention time t_{R} is given in minutes and the molecular mass is always given as [M+H]⁺ for the LC-MS analyses. Standard measurements were made on a Waters Micromass LC-MS system.

### Examples 6 - 15

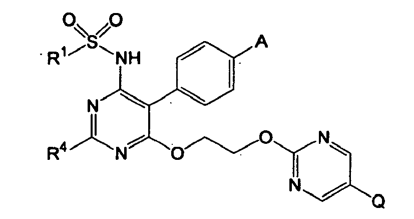

| **Ex. Nr** | **R¹:** | **R⁴:** | **A** | **Q** | **LC-MS** | |
|---|---|---|---|---|---|---|
| 6 | 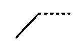 | 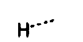 | Br--- | Br ----- | t_{R}:4.91 | [M+H]⁺: 559.80 |
| 7 | 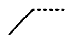 | 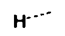 | Br--- | H₃CO ---- | t_{R}:4.53 | [M+H]⁺: 511.95 |

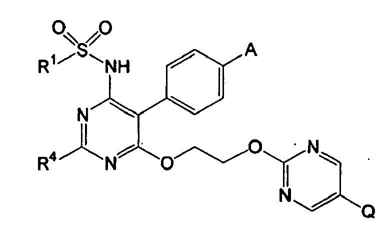

| **Ex. Nr** | **R¹:** | **R⁴:** | **A** | **Q** | **LC-MS** | |
|---|---|---|---|---|---|---|
| 8 | 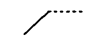 | 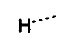 | Cl----- | Br----- | t_{R}: 4.93 | [M+H]⁺: 515.96 |
| 9 | 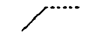 | 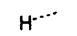 | Cl----- | H₃CO---- | t_{R}: 4.48 | [M+H]⁺: 466.15 |
| 10 | 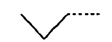 | 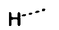 | Br----- | Br----- | t_{R}: 5.21 | [M+H]⁺: 573.86 |
| 11 | 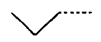 | 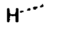 | Br----- | H₃CO---- | t_{R}: 4.80 | [M+H]⁺: 525.99 |
| 12 | 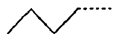 | 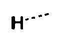 | H₃C---- | Br----- | t_{R}: 6.11 | [M+H]⁺: 524.06 |
| 13 | 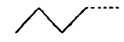 | 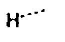 | H₃C---- | H₃CO---- | t_{R}: 4.94 | [M+H]⁺: 474.23 |

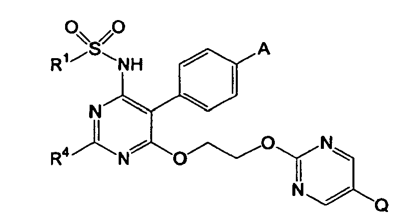

| Ex. Nr | R¹: | R⁴: | A | Q | LC-MS | |
|---|---|---|---|---|---|---|
| 14 | 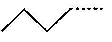 | 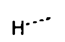 | Br----- | Br----- | t_{R}: 5.48 | [M+H]⁺: 588.13 |
| 15 | 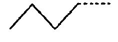 | 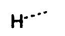 | Br----- | H₃CO---- | t_{R}: 5.06 | [M+H]⁺: 539.28 |

### Example 16:

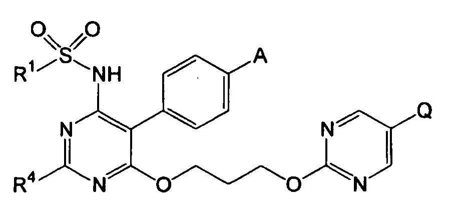

| **R¹:** | **R⁴:** | | **A** | **Q** |
|---|---|---|---|---|
| 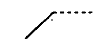 | 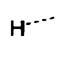 | 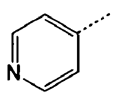 | H ---- | Br ----- |
| 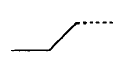 | 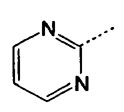 | | H₃C ---- | Cl----- |
| 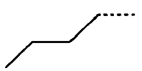 | | 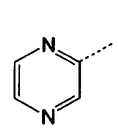 | Cl----- | H₃C ---- |
| | | | Br----- | H₃CO---- |
| | | | | H ---- |

## Claims

1. A compound of the formula VI: 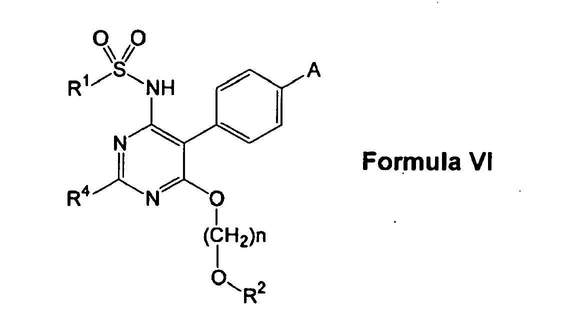 wherein
**R¹** represents ethyl; propyl; butyl;
**R²** represents aryl; heteroaryl;
**R⁴** represents hydrogen or heteroaryl;
**A** represents hydrogen; methyl; ethyl; chlorine; bromine;
and n represents the integers 2; 3;
it being understood that:
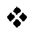 the expression "aryl" represents unsubstituted as well as mono-, di- or tri-substituted phenyl or naphthyl rings which may be substituted with aryl; halogen; hydroxy; alkyl of 1 to 7 carbon atoms; alkenyl of 2 to 7 carbon atoms; alkynyl of 2 to 7 carbon atoms; alkoxy of 1 to 7 carbon atoms; alkenyloxy of 2 to 7 carbon atoms; alkynyl-alkoxy wherein the alkynyl is an alkynyl of 2 to 7 carbon atoms and the alkoxy is an alkoxy of 1 to 7 carbon atoms; alkenylen of 1 to 7 carbon atoms; allyloxy, vinyloxy or propenyloxy; methylendioxy or ethylendioxy forming with the phenyl ring a five- or six-membered ring; hydroxy-alkyl of 1 to 7 carbon atoms; hydroxy-alkenyl of 2 to 7 carbon atoms; hydroxy-alkyl-alkynyl wherein the alkyl is an alkyl of 1 to 7 carbon atoms and the alkynyl is an alkynyl of 2 to 7 carbon atoms; alkoxy-alkyl wherein the alkoxy is an alkoxy of 1 to 7 carbon atoms and the alkyl is an alkyl of 1 to 7 carbon atoms; alkoxy-alkoxy wherein each alkoxy is an alkoxy of 1 to 7 carbon atoms; trifluoromethyl; trifluoromethoxy; cycloalkyl of 3 to 7 carbon atoms optionally substituted by groups selected from alkyl of 1 to 7 carbon atoms, hydroky-alkyl of 1 to 7 carbon atoms, amino-alkyl of 1 to 7 carbon atoms and alkoxy-alkyl wherein the alkoxy is an alkoxy of 1 to 7 carbon atoms and the alkyl is an alkyl of 1 to 7 carbon atoms; hydroxy-cycloalkyl; heterocyclyl; heteroaryl;
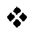 the expression "heteroaryl" means six-membered aromatic rings containing one to four nitrogen atoms, benzofused six-membered aromatic rings containing one to three nitrogen atoms, five-membered aromatic rings containing one oxygen or one nitrogen or one sulfur atom, benzofused five-membered aromatic rings containing one oxygen or one nitrogen or one sulfur atom, five membered aromatic rings containing an oxygen and nitrogen atom and benzo fused derivatives thereof, five-membered aromatic rings containing a sulfur and a nitrogen atom and benzo fused derivatives thereof, five-membered aromatic rings containing two nitrogen atoms and benzo fused derivatives thereof, five membered aromatic rings containing three nitrogen atoms and benzo fused derivatives thereof or the tetrazolyl ring, whereby such rings may be substituted with alkyl of 1 to 7 carbon atoms, alkenyl of 2 to 7 carbon atoms, amino, amino-alkyl of 1 to 7 carbon atoms, halogen, hydroxy, alkoxy of 1 to 7 carbon atoms, trifluoromethoxy, trifluoromethyl, carboxyl, carboxamidyl, thioamidyl, amidinyl, alkoxy-carbonyl wherein the alkoxy is an alkoxy of 1 to 7 carbon atoms, cyano, hydroxy-alkyl of 1 to 7 carbon atoms, alkoxy-alkyl wherein the alkoxy is an alkoxy of 1 to 7 carbon atoms and the alkyl is an alkyl of 1 to 7 carbon atoms or another heteroaryl- or heterocyclyl-ring;
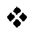 the expression "heterocyclyl" means saturated or unsaturated (but not aromatic), four, five-, six- or seven-membered rings containing one or two nitrogen, oxygen or sulfur atoms which may be the same or different and which rings may be adequately substituted with alkyl of 1 to 7 carbon atoms, alkoxy of 1 to 7 carbon atoms;
or an optically pure enantiomer or diastereomer, a mixture of enantiomers or diastereomers, a diastereomeric racemate, a mixture of diastereomeric racemates, a meso-form or a pharmaceutically acceptable salt thereof.

2. A compound according to claim 1, **characterized in that** it is compound of the formula VII 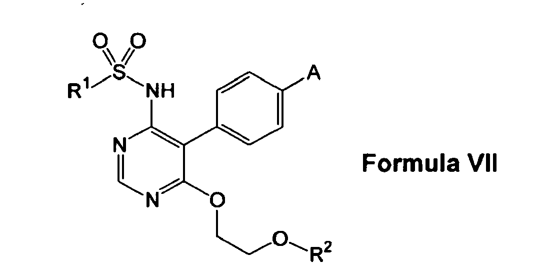 wherein
**R¹** represents ethyl; propyl; butyl;
**R²** represents heteroaryl;
**A** represents methyl; chlorine; bromine;
or an optically pure enantiomer or diastereomer, a mixture of enantiomers or diastereomers, a diastereomeric racemate, a mixture of diastereomeric racemates, a meso-form or a pharmaceutically acceptable salt thereof.

3. A compound according to claim 1, selected from the following:
Ethanesulfonic acid {6-[2-(5-bromo-pyrimidin-2-yloxy)-ethoxy]-5-p-tolyl-pyrimidin-4-yl}-amide;
n-Propanesulfonic acid {6-[2-(5-bromo-pyrimidin-2-yloxy)-ethoxy]-5-p-tolyl-pyrimidin-4-yl}-amide;
Ethanesulfonic acid [6-[2-(5-bromo-pyrimidin-2-yloxy)-ethoxy]-5-(4-chlorophenyl)-pyrimidin-4-yl]-amide;
n-Propanesulfonic acid [6-[2-(5-bromo-pyrimidin-2-yloxy)-ethoxy]-5-(4-chlorophenyl)-pyrimidin-4-yl]-amide;
Ethanesulfonic acid {5-(4-bromo-phenyl)-6-[2-(5-bromo-pyrimidin-2-yloxy)-ethoxy]-pyrimidin-4-yl}-amide;
n-Propanesulfonic acid {5-(4-bromo-phenyl)-6-[2-(5-bromo-pyrimidin-2-yloxy)-ethoxy]-pyrimidin-4-yl}-amide;
Ethanesulfonic acid [6-[2-(5-bromo-pyrimidin-2-yloxy)-ethoxy]-5-(2-methoxyphenoxy)-[2,2']bipyrimidinyl-4-yl]-amide;
N-[6-[2-(5-Bromo-pyrimidin-2-yloxy)-ethoxy]-5-p-tolyl-pyrimidin-4-yl]-methanesulfonamide;
or a pharmaceutically acceptable salt thereof.

4. A compound according to claim 1, selected from the following:
N-[5-(4-Bromo-phenyl)-6-[2-(5-bromo-pyrimidin-2-yloxy)-ethoxy]-pyrimidin-4-yl]-methanesulfonamide;
Ethanesulfonic acid [5-(4-bromo-phenyl)-6-[2-(5-bromo-pyrimidin-2-yloxy)-ethoxy]-pyrimidin-4-yl]-amide;
Propane-1-sulfonic acid [5-(4-bromo-phenyl)-6-[2-(5-bromo-pyrimidin-2-yloxy)-ethoxy]-pyrimidin-4-yl]-amide;
or a pharmaceutically acceptable salt thereof.

5. A compound of any one of claims 1 to 4 for use as medicament for the treatment of disorders which are associated with a role of endothelin.

6. A compound of any one of claims 1 to 4 for use as medicament for the treatment of circulatory disorders which are associated with a role of endothelin.

7. A compound of any one of the claims 1 to 4 for use as medicament for the treatment of inflammatory disorders which are associated with a role of endothelin.

8. A compound of any one of the claims 1 to 4 for use as medicament for the treatment of proliferative disorders which are associated with a role of endothelin.

9. A compound of any one of the claims 1 to 4 for use as medicaments for the treatment of hypertension, coronary diseases, cardiac insufficiency, renal and myocardial ischemia, renal failure, cerebral ischemia, dementia, migraine, subarachnoidal hemorrhage, Raynaud's syndrome, portal hypertension, pulmonary hypertension, atherosclerosis, prevention of restenosis after balloon or stent angioplasty, inflammation, pulmonary fibrosis, connective tissue diseases, stomach and duodenal ulcer, digital ulcer, cancer, prostatic hypertrophy, erectile dysfunction, hearing loss, amaurosis, chronic bronchitis, asthma, gram negative septicemia, shock, sickle cell anemia, glomerulonephritis, renal colic, glaucoma, complications of vascular or cardiac surgery or after organ transplantation, and complications of cyclosporin, the therapy and prophylaxis of diabetic complications.

10. A compound of any one of the claims 1 to 4 for use as medicament for the treatment of disorders which are associated with a role of endothelin, and require mixed ET_{A} and ET_{B} blocking for treatment.

11. A compound of any one of the claims 1 to 4 for use as medicament for the treatment of disorders which are associated with a role of endothelin, and require selective ET_{A} blocking for treatment.

12. A compound of any one of the claims 1 to 4 for use as medicament for the treatment of disorders which are associated with a role of endothelin, and require selective ET_{B} blocking for treatment.

13. The use of one or more compounds of any one of claims 1 to 4 as active ingredients for the production of pharmaceutical compositions for the treatment of disorders associated with a role of endothelin.

14. The use of claim 13, wherein the disorders associated with a role of endothelin are circulatory disorders.

15. The use of claim 13, wherein the disorders associated with a role of endothelin are inflammatory disorders.

16. The use of claim 13, wherein the disorders associated with a role of endothelin are proliferative disorders.

17. The use of claim 13, wherein the disorders associated with a role of endothelin are hypertension, coronary diseases, cardiac insufficiency, renal and myocardial ischemia, renal failure, cerebral ischemia, dementia, migraine, subarachnoidal hemorrhage, Raynaud's syndrome, portal hypertension, pulmonary hypertension, atherosclerosis, prevention of restenosis after balloon or stent angioplasty, inflammation, pulmonary fibrosis, connective tissue diseases, stomach and duodenal ulcer, digital ulcer, cancer, melanoma, prostatic cancer, prostatic hypertrophy, erectile dysfunction, hearing loss, amaurosis, chronic bronchitis, asthma, gram negative septicemia, shock, sickle cell anemia, glomerulonephritis, renal colic, glaucoma, therapy and prophylaxis of diabetic complications, complications of vascular or cardiac surgery or after organ transplantation, and complications of cyclosporin.

18. The use of claim 17, wherein the compound(s) of claims 1 to 4 is (are) for use in combination with one or more other therapeutically useful substances.

19. The use of claim 18, wherein the therapeutically useful substance(s) is (are) selected from the group consisting of α- and β-blockers, vasodilators, calcium-antagonists, ACE-inhibitors, potassium activators, angiotensin II receptor antagonists, diuretics, sympatholitics and other therapeutics which serve to treat high blood pressure or any cardiac disorders.

20. A process for the manufacture of pharmaceutical compositions for the treatment of disorders associated with a role of endothelin containing one or more compounds as claimed in any one of claims 1 to 4 as active ingredients which process comprises mixing one or more active ingredients with pharmaceutically acceptable excipients in a manner known per se.

## Patentansprüche

1. Verbindung der Formel VI: 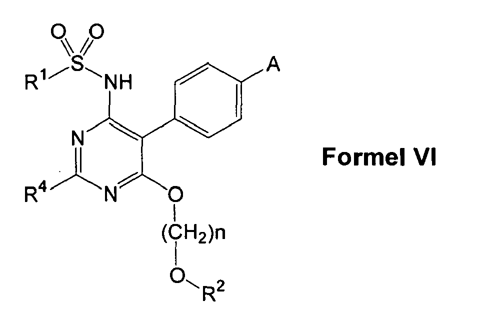 wobei
**R¹** Ethyl, Propyl, Butyl repräsentiert;
**R²** Aryl, Heteroaryl repräsentiert;
**R⁴** Wasserstoff oder Heteroaryl repräsentiert;
**A** Wasserstoff, Methyl, Ethyl, Chlor, Brom repräsentiert;
und n die ganzen Zahlen 2, 3 repräsentiert;
wobei zu verstehen ist, dass:
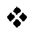 der Begriff "Aryl" nichtsubstituierte sowie mono-, di- oder tri-substituierte Phenyl-oder Naphthyl-Ringe repräsentiert, die substituiert sein können durch Aryl; Halogen; Hydroxy; Alkyl mit 1 bis 7 Kohlenstoffatomen; Alkenyl mit 2 bis 7 Kohlenstoffatomen; Alkynyl mit 2 bis 7 Kohlenstoffatomen; Alkoxy mit 1 bis 7 Kohlenstoffatomen; Alkenyloxy mit 2 bis 7 Kohlenstoffatomen; Alkynyl-Alkoxy, wobei das Alkynyl ein Alkynyl mit 2 bis 7 Kohlenstoffatomen ist und das Alkoxy ein Alkoxy mit 1 bis 7 Kohlenstoffatomen ist; Alkenylen mit 1 bis 7 Kohlenstoffatomen; Allyloxy, Vinyloxy oder Propenyloxy; Methylendioxy oder Ethylendioxy, das mit dem Phenylring einen fünf- oder sechsgliedrigen Ring bildet; Hydroxy-Alkyl mit 1 bis 7 Kohlenstoffatomen; Hydroxy-Alkenyl mit 2 bis 7 Kohlenstoffatomen; Hydroxy-Alkyl-Alkynyl, wobei das Alkyl ein Alkyl mit 1 bis 7 Kohlenstoffatomen ist und das Alkynyl ein Alkynyl mit 2 bis 7 Kohlenstoffatomen ist; Alkoxy-Alkyl, wobei das Alkoxy ein Alkoxy mit 1 bis 7 Kohlenstoffatomen ist und das Alkyl ein Alkyl mit 1 bis 7 Kohlenstoffatomen ist; Alkoxy-Alkoxy, wobei jedes Alkoxy ein Alkoxy mit 1 bis 7 Kohlenstoffatomen ist; Trifluormethyl; Trifluormethoxy; Cycloalkyl mit 3 bis 7 Kohlenstoffatomen, optional substituiert durch Gruppen, die ausgewählt sind aus Alkyl mit 1 bis 7 Kohlenstoffatomen, Hydroxy-Alkyl mit 1 bis 7 Kohlenstoffatomen, Amino-Alkyl mit 1 bis 7 Kohlenstoffatomen und Alkoxy-Alkyl, wobei das Alkoxy ein Alkoxy mit 1 bis 7 Kohlenstoffatomen ist und das Alkyl ein Alkyl mit 1 bis 7 Kohlenstoffatomen ist; Hydroxy-Cycloalkyl; Heterocyclyl; Heteroaryl;
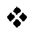 der Begriff "Heteroaryl" sich auf Folgendes bezieht: sechsgliedrige aromatische Ringe, die ein bis vier Stickstoffatome enthalten, benzokondensierte sechsgliedrige aromatische Ringe, die ein bis drei Stickstoffatome enthalten, fünfgliedrige aromatische Ringe, die ein Sauerstoff- oder ein Stickstoff- oder ein Schwefelatom enthalten, benzokondensierte fünfgliedrige aromatische Ringe, die ein Sauerstoff- oder ein Stickstoff- oder ein Schwefelatom enthalten, fünfgliedrige aromatische Ringe, die ein Sauerstoff- und Stickstoffatom enthalten, und benzokondensierte Derivate davon, fünfgliedrige aromatische Ringe, die ein Schwefel- und ein Stickstoffatom enthalten, und benzokondensierte Derivate davon, fünfgliedrige aromatische Ringe, die zwei Stickstoffatome enthalten, und benzokondensierte Derivate davon, fünfgliedrige aromatische Ringe, die drei Stickstoffatome enthalten, und benzokondensierte Derivate davon, oder den Tetrazolylring, wobei solche Ringe substituiert sein können durch Alkyl mit 1 bis 7 Kohlenstoffatomen, Alkenyl mit 2 bis 7 Kohlenstoffatomen, Amino, Amino-Alkyl mit 1 bis 7 Kohlenstoffatomen, Halogen, Hydroxy, Alkoxy mit 1 bis 7 Kohlenstoffatomen, Trifluormethoxy, Trifluormethyl, Carboxyl, Carboxamidyl, Thioamidyl, Amidinyl, Alkoxy-Carbonyl, wobei das Alkoxy ein Alkoxy mit 1 bis 7 Kohlenstoffatomen ist, Cyano, Hydroxy-Alkyl mit 1 bis 7 Kohlenstoffatomen, Alkoxy-Alkyl, wobei das Alkoxy ein Alkoxy mit 1 bis 7 Kohlenstoffatomen ist und das Alkyl ein Alkyl mit 1 bis 7 Kohlenstoffatomen ist, oder einen anderen Heteroaryl- oder Heterocyclylring;
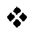 der Begriff "Heterocyclyl" Folgendes bedeutet: gesättigte oder ungesättigte (aber nicht aromatische), vier-, fünf-, sechs- oder siebengliedrige Ringe, die ein oder zwei Stickstoff-, Sauerstoff- oder Schwefelatome enthalten, die gleich oder unterschiedlich sein können, wobei die Ringe angemessen substituiert sein können durch Alkyl mit 1 bis 7 Kohlenstoffatomen, Alkoxy mit 1 bis 7 Kohlenstoffatomen;
oder ein optisch reines Enantiomer oder Diastereomer, ein Gemisch von Enantiomeren oder Diastereomeren, eine diastereomere Racemate, ein Gemisch von diastereomeren Racematen, eine meso-Form oder ein pharmazeutisch akzeptables Salz davon.

2. Verbindung nach Anspruch 1, **dadurch gekennzeichnet, dass** sie eine Verbindung der Formel VII ist 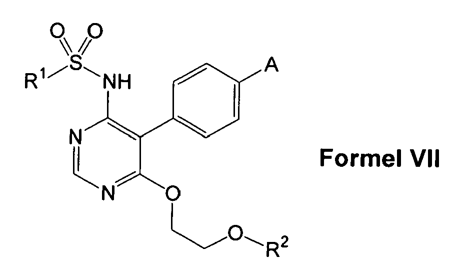 wobei
**R¹** Ethyl, Propyl, Butyl repräsentiert;
**R²** Heteroaryl repräsentiert;
**A** Methyl, Chlor, Brom repräsentiert;
oder ein optisch reines Enantiomer oder Diastereomer, ein Gemisch von Enantiomeren oder Diastereomeren, eine diastereomere Racemate, ein Gemisch von diastereomeren Racematen, eine meso-Form oder ein pharmazeutisch akzeptables Salz davon.

3. Verbindung nach Anspruch 1, ausgewählt aus den Folgenden:
Ethansulfonsäure-{6-[2-(5-brom-pyrimidin-2-yloxy)-ethoxy]-5-p-tolyl-pyrimidin-4-yl}-amid;
n-Propansulfonsäure-{6-[2-(5-brom-pyrimidin-2-yloxy)-ethoxy]-5-p-tolyl-pyrimidin-4-yl}-amid;
Ethansulfonsäure-[6-[2-(5-brom-pyrimidin-2-yloxy)-ethoxy]-5-(4-chlor-phenyl)-pyrimidin-4-yl]-amid;
n-Propansulfonsäure-[6-[2-(5-brom-pyrimidin-2-yloxy)-ethoxy]-5-(4-chlorphenyl)-pyrimidin-4-yl]-amid;
Ethansulfonsäure-{5-(4-brom-phenyl)-6-[2-(5-brom-pyrimidin-2-yloxy)-ethoxy]-pyrimidin-4-yl}-amid;
n-Propansulfonsäure-{5-(4-brom-phenyl)-6-[2-(5-brom-pyrimidin-2-yloxy)-ethoxy]-pyrimidin-4-yl}-amid;
Ethansulfonsäure-[6-[2-(5-brom-pyrimidin-2-yloxy)-ethoxy]-5-(2-methoxyphenoxy)-[2,2']bipyrimidinyl-4-yl]-amid;
N-[6-[2-(5-Brom-pyrimidin-2-yloxy)-ethoxy]-5-p-tolyl-pyrimidin-4-yl]-methansulfonamid;
oder ein pharmazeutisch akzeptables Salz davon.

4. Verbindung nach Anspruch 1, ausgewählt aus den Folgenden:
N-[5-(4-Brom-phenyl)-6-[2-(5-brom-pyrimidin-2-yloxy)-ethoxy]-pyrimidin-4-yl]-methansulfonamid;
Ethansulfonsäure-[5-(4-brom-phenyl)-6-[2-(5-brom-pyrimidin-2-yloxy)-ethoxy]-pyrimidin-4-yl]-amid;
Propan-1-sulfonsäure-[5-(4-brom-phenyl)-6-[2-(5-brom-pyrimidin-2-yloxy)-ethoxy]-pyrimidin-4-yl]-amid;
oder ein pharmazeutisch akzeptables Salz davon.

5. Verbindung nach einem der Ansprüche 1 bis 4 zur Verwendung als Medikament zur Behandlung von Störungen, die mit einer Rolle von Endothelin zusammenhängen.

6. Verbindung nach einem der Ansprüche 1 bis 4 zur Verwendung als Medikament zur Behandlung von Kreislaufstörungen, die mit einer Rolle von Endothelin zusammenhängen.

7. Verbindung nach einem der Ansprüche 1 bis 4 zur Verwendung als Medikament zur Behandlung von Entzündungsstörungen, die mit einer Rolle von Endothelin zusammenhängen.

8. Verbindung nach einem der Ansprüche 1 bis 4 zur Verwendung als Medikament zur Behandlung von Proliferationsstörungen, die mit einer Rolle von Endothelin zusammenhängen.

9. Verbindung nach einem der Ansprüche 1 bis 4 zur Verwendung als Medikament zur Behandlung von Hypertonie, Koronarkrankheiten, Herzinsuffizienz, Nieren- und Myokardischämie, Nierenversagen, zerebraler Ischämie, Demenz, Migräne, Subarachnoidalblutung, dem Raynaudschen Syndrom, portaler Hypertonie, pulmonaler Hypertonie, Atherosklerose, zur Vorbeugung gegen Restenose nach einer Ballon- oder Stent-Angioplastie, Inflammation, Lungenfibrose, Bindegewebserkrankungen, Magen- und Zwölffingerdarmgeschwür, Fingergeschwür, Krebs, Prostatahypertrophie, erektile Dysfunktion, Hörverlust, Amaurose, chronische Bronchitis, Asthma, gramnegative Septikämie, Schock, Sichelzellenanämie, Glomerulonephritis, Nierenkolik, Glaukom, bei Komplikationen in der Gefäß- oder Herzchirurgie oder nach Organtransplantationen, Cyclosporin-Komplikationen, zur Therapie und Prophylaxe von diabetischen Komplikationen.

10. Verbindung nach einem der Ansprüche 1 bis 4 zur Verwendung als Medikament zur Behandlung von Störungen, die mit einer Rolle von Endothelin zusammenhängen und zur Behandlung eine gemischte ET_{A}- und ET_{B}-Blockierung erfordern.

11. Verbindung nach einem der Ansprüche 1 bis 4 zur Verwendung als Medikament zur Behandlung von Störungen, die mit einer Rolle von Endothelin zusammenhängen und zur Behandlung eine selektive ET_{A}-Blockierung erfordern.

12. Verbindung nach einem der Ansprüche 1 bis 4 zur Verwendung als Medikament zur Behandlung von Störungen, die mit einer Rolle von Endothelin zusammenhängen und zur Behandlung eine selektive ET_{B}-Blockierung erfordern.

13. Verwendung von einer oder mehreren Verbindungen nach einem der Ansprüche 1 bis 4 als aktive Bestandteile zur Herstellung pharmazeutischer Zusammensetzungen zur Behandlung von Störungen, die mit einer Rolle von Endothelin zusammenhängen.

14. Verwendung nach Anspruch 13, wobei die Störungen, die mit einer Rolle von Endothelin zusammenhängen, Kreislaufstörungen sind.

15. Verwendung nach Anspruch 13, wobei die Störungen, die mit einer Rolle von Endothelin zusammenhängen, Entzündungsstörungen sind.

16. Verwendung nach Anspruch 13, wobei die Störungen, die mit einer Rolle von Endothelin zusammenhängen, Proliferationsstörungen sind.

17. Verwendung nach Anspruch 13, wobei die Störungen, die mit einer Rolle von Endothelin zusammenhängen, die Folgenden sind: Hypertonie, Koronarkrankheiten, Herzinsuffizienz, Nieren- und Myokardischämie, Nierenversagen, zerebrale Ischämie, Demenz, Migräne, Subarachnoidalblutung, Raynaudsches Syndrom, portale Hypertonie, pulmonale Hypertonie, Atherosklerose, Vorbeugung gegen Restenose nach einer Ballon- oder Stent-Angioplastie, Inflammation, Lungenfibrose, Bindegewebserkrankungen, Magen- und Zwölffingerdarmgeschwür, Fingergeschwür, Krebs, Melanom, Prostatakrebs, Prostatahypertrophie, erektile Dysfunktion, Hörverlust, Amaurose, chronische Bronchitis, Asthma, gramnegative Septikämie, Schock, Sichelzellenanämie, Glomerulonephritis, Nierenkolik, Glaukom, Therapie und Prophylaxe von diabetischen Komplikationen, Komplikationen in der Gefäß- oder Herzchirurgie oder nach Organtransplantationen und Cyclosporin-Komplikationen.

18. Verwendung nach Anspruch 17, wobei die Verbindung(en) aus den Ansprüchen 1 bis 4 zur Verwendung in Kombination mit einer oder mehreren therapeutisch nützlichen Substanzen vorgesehen ist (sind).

19. Verwendung nach Anspruch 18, wobei die therapeutisch nützliche(n) Substanz(en) ausgewählt ist (sind) aus der Gruppe bestehend aus α- und β-Blockern, Vasodilatatoren, Calciumantagonisten, ACE-Inhibitoren, Kaliumaktivatoren, Angiotensin-II-Rezeptor-Antagonisten, Diuretika, Sympathikolytika und anderen Therapeutika, die zur Behandlung von hohem Blutdruck oder beliebigen Herzstörungen vorgesehen sind.

20. Verfahren zur Herstellung pharmazeutischer Zusammensetzungen zur Behandlung von Störungen, die mit einer Rolle von Endothelin zusammenhängen, die eine oder mehrere Verbindungen nach einem der Ansprüche 1 bis 4 als aktive Bestandteile enthalten, wobei das Verfahren das Vermischen von einem oder mehreren aktiven Bestandteilen mit pharmazeutisch akzeptablen Exzipienten in einer per se bekannten Weise beinhaltet.

## Revendications

1. Composé de la formule VI 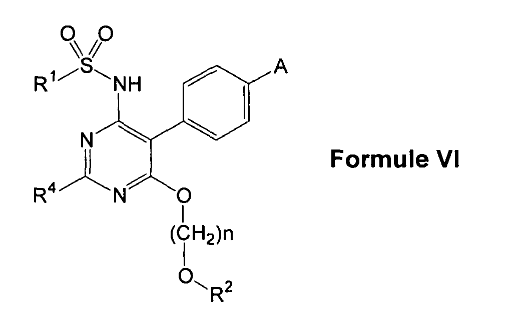 où
**R¹** représente un groupement éthyle; propyle; butyle;
**R²** représente un groupement aryle; hétéroaryle;
**R⁴** représente l'hydrogène ou un groupement hétéroaryle;
**A** représente un hydrogène; un groupement méthyle; éthyle; un chlore; un brome;
et n représente les entiers 2; 3;
étant donné qu'il est entendu que:
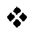 l'expression "aryle" représente des cycles phényle ou naphtyle non substitués ainsi que mono-, di- ou tri-substitués qui peuvent être substituées par des groupements aryle, halogène, hydroxy, alkyles ayant de 1 à 7 atomes de carbone, alcényle ayant de 2 à 7 atomes de carbone , alcynyle ayant de 2 à 7 atomes de carbone, alkoxy ayant de 1 à 7 atomes de carbone, alcényloxy ayant de 2 à 7 atomes de carbone, alcynyl-alkoxy où le groupement alcynyle est un alcynyle de 2 à 7 atomes de carbone et le groupement alkoxy est un alkoxy ayant de 1 à 7 atomes de carbone, alcénylène ayant de 1 à 7 atomes de carbone, allyloxy, vinyloxy ou propényloxy, méthylènedioxy ou éthylènedioxy formant avec le cycle phényle un cycle à cinq ou six membres, hydroxy-alkyle ayant de 1 à 7 atomes de carbone, hydroxy-alcényle ayant de 2 à 7 atomes de carbone, hydroxy-alkyl-alcynyle où le groupement alkyle est un alkyle ayant de 1 à 7 atomes de carbone et le groupement alcynyle est un alcynyle ayant de 2 à 7 atomes de carbone, alkoxy-alkyle où le groupement alkoxy est un alkoxy ayant de 1 à 7 atomes de carbone et le groupement alkyle est un alkyle ayant de 1 à 7 atomes de carbone, alkoxy-alkoxy où chaque groupement alkoxy est un alkoxy ayant de 1 à 7 atomes de carbone, trifluorométhyle, trifluorométhoxy, cycloalkyle ayant de 3 à 7 atomes de carbone, substitués en option par des groupements sélectionnés parmi les groupements alkyle ayant de 1 à 7 atomes de carbone , hydroxy-alkyle ayant de 1 à 7 atomes de carbone, amino-alkyle ayant de 1 à 7 atomes de carbone et alkoxy-alkyle où le groupement alkoxy est un alkoxy ayant de 1 à 7 atomes de carbone et le groupement alkyle est un alkyle de 1 à 7 atomes de carbone, hydroxy-cycloalkyle, hétérocyclyle, hétéroaryle.
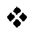 l'expression "hétéroaryle" veut dire des cycles aromatiques à six membres contenant d'un à quatre atomes d'azote, des cycles aromatiques à six membres accolés benzo contenant d'un à trois atomes d'azote, des cycles aromatiques à cinq membres contenant un atome d'oxygène ou d'azote ou de soufre, des cycles aromatiques à cinq membres accolés benzo contenant un atome d'oxygène ou d'azote ou de soufre, des cycles aromatiques à cinq membres contenant un atome d'oxygène et une atome d'azote et des dérivés accolés benzo de ces derniers, des cycles aromatiques à cinq membres contenant un atome de soufre et un atome d'azote et des dérivés accolés benzo de ces derniers, des cycles aromatiques à cinq membres contenant deux atomes d'azote et des dérivés accolés benzo de ces derniers, des cycles aromatiques à cinq membres contenant trois atomes d'azote et des dérivés accolés benzo de ces derniers ou le cycle tétrazolyle; des cycles de ce genre pouvant être substitués par des groupements alkyles ayant de 1 à 7 atomes de carbone, alcényle ayant de 2 à 7 atomes de carbone, amino, amino-alkyle ayant de 1 à 7 atomes de carbone, halogène, hydroxy, alkoxy de 1 à 7 atomes de carbone, trifluorométhoxy, trifluorométhyle, carboxyle, carboxamidyle, thioamidyle, amidinyle, alkoxy-carbonyle où le groupement alkoxy est un alkoxy ayant de 1 à 7 atomes de carbone, cyano, hydroxy-alkyle ayant de 1 à 7 atomes de carbone, alkoxy-alkyle où le groupement alkoxy est un alkoxy ayant de 1 à 7 atomes de carbone et le groupement alkyle est un alkyle ayant de 1 à 7 atomes de carbone ou un autre cycle hétéroaryle ou hétérocyclyle.
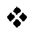 l'expression "hétérocyclyle" veut dire des cycles à quatre, cinq, six ou sept membres, saturés ou non saturés (mains non aromatiques), contenant un ou deux atomes d'azote, d'oxygène ou de soufre qui peuvent être identiques ou différents et dont les cycles peuvent être substitués d'une manière adéquate à l'aide d'un groupement alkyle ayant de 1 à 7 atomes de carbone, d'un groupement alkoxy ayant de 1 à 7 atomes de carbone;
ou un énantiomère ou un diastéréoisomère optiquement pur, un mélange d'énantiomères ou de diastéréoisomères, un racémate diastéréoisomère, un mélange de racémates diastéréoisomères, une forme méso ou un sel acceptable du point de vue pharmaceutique de ces derniers.

2. Composé selon la revendication 1, **caractérisé en ce qu'**il s'agit d'un composé de la formule VII 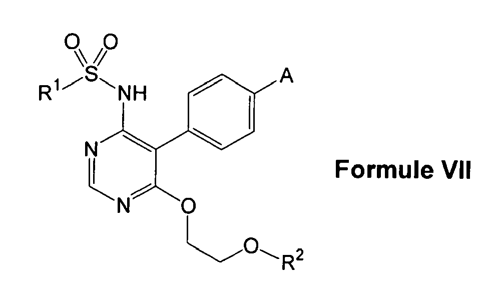 où
**R¹** représente un groupement éthyle; propyle; butyle;
**R²** représente un groupement hétéroaryle;
**A** représente un groupement méthyle; un chlore; un brome;
ou un énantiomère ou un diastéréoisomère optiquement pur, un mélange d'énantiomères ou de diastéréoisomères, un racémate diastéréoisomère, un mélange de racémates diastéréoisomères, une forme méso ou un sel acceptable du point de vue pharmaceutique de ces derniers.

3. Composé selon la revendication 1, sélectionné parmi les composé suivants:
le {6-[2-(5-bromo-pyrimidin-2-yloxy)-éthoxy]-5-p-tolyl-pyrimidin-4-yl}-amide de l'acide éthane-sulfonique;
le {6-[2-(5-bromo-pyrimidin-2-yloxy)-éthoxy]-5-p-tolyl-pyrimidin-4-yl}-amide de l'acide n-propanesulfonique;
le [6-[2-(5-bromo-pyrimidin-2-yloxy)-éthoxy]-5-(4-chloro-phényl)-pyrimidin-4-yl]-amide de l'acide éthane-sulfonique;
le [6-[2-(5-bromo-pyrimidin-2-yloxy)-éthoxy]-5-(4-chloro-phényl)-pyrimidin-4-yl]-amide de l'acide n-propanesulfonique;
le {5-(4-bromo-phényl)-6-[2-(5-bromo-pyrimidin-2-yloxy)-éthoxy]-pyrimidin-4-yl}-amide de l'acide éthane-sulfonique;
le {5-(4-bromo-phényl)-6-[2-(5-bromo-pyrimidin-2-yloxy)-éthoxy]-pyrimidin-4-yl}-amide de l'acide n-propanesulfonique;
le [6-[2-(5-bromo-pyrimidin-2-yloxy)-éthoxy]-5-(2-méthoxy-phénoxy)-[2,2']bipyrimidinyl-4-yl]-amide de l'acide éthane-sulfonique;
le N-[6-[2-(5-bromo-pyrimidin-2-yloxy)-éthoxy]-5-p-tolyl-pyrimidin-4-yl]-méthanesulfonamide;
ou un sel, acceptable du point de vue pharmaceutique, de ce dernier.

4. Composé selon la revendication 1, sélectionné parmi les composé suivants:
le N-[5-(4-bromo-phényl)-6-[2-(5-bromo-pyrimidin-2-yloxy)-éthoxy]-pyrimidin-4-yl]-méthanesulfonamide;
le {5-(4-bromo-phényl)-6-[2-(5-bromo-pyrimidin-2-yloxy)-éthoxy]-pyrimidin-4-yl}-amide de l'acide éthane-sulfonique;
le [5-(4-bromo-phényl)-6-[2-(5-bromo-pyrimidin-2-yloxy)-éthoxy]-pyrimidin-4-yl]-amide de l'acide propane-1-sulfonique.
ou un sel, acceptable du point de vue pharmaceutique, de ce dernier.

5. Composé selon l'une quelconque des revendications 1 à 4 pour utilisation en tant que médicament pour le traitement de troubles qui sont associés à un rôle de l'endothéline.

6. Composé selon l'une quelconque des revendications 1 à 4 pour utilisation en tant que médicament pour le traitement de troubles de la circulation qui sont associés à un rôle de l'endothéline.

7. Composé selon l'une quelconque des revendications 1 à 4 pour utilisation en tant que médicament pour le traitement de troubles inflammatoires qui sont associés à un rôle de l'endothéline.

8. Composé selon l'une quelconque des revendications 1 à 4 pour utilisation en tant que médicament pour le traitement de troubles de la prolifération qui sont associés à un rôle de l'endothéline.

9. Composé selon l'une quelconque des revendications 1 à 4 pour utilisation en tant que médicaments pour le traitement de l'hypertension, des maladies coronaires, de l'insuffisance cardiaque, de l'ischémie rénale et myocardique, de la défaillance rénale, de l'ischémie cérébrale, de la démence, de la migraine, de l'hémorragie sous-arachnoïdienne, du syndrome de Raynaud, de l'hypertension portale, de l'hypertension pulmonaire, de l'athérosclérose, de la prévention de la resténose après angioplastie à ballon ou à stent, de l'inflammation, de la fibrose pulmonaire, des maladies des tissus connectifs, de l'ulcère d'estomac et de l'ulcère du duodénum, de l'ulcère digital, du cancer, de l'hypertrophie prostatique, de la dysfonction érectile, de la perte auditive, de l'amaurose, de la bronchite chronique, de l'asthme, de la septicémie gramme-négative, des chocs, de la drépanocytose, de la glomérulonéphrite, de la colique rénale, du glaucome, des complications de la chirurgie vasculaire ou cardiaque ou après transplantation d'organes et de complications dues à la cyclosporine, pour la thérapie et la prophylaxie des complications diabétiques.

10. Composé selon l'une quelconque des revendications 1 à 4 pour utilisation en tant que médicament pour le traitement de troubles qui sont associés à un rôle de l'endothéline et exigent un blocage ET_{A} et ET_{B} mixte pour le traitement.

11. Composé selon l'une quelconque des revendications 1 à 4 pour utilisation en tant que médicament pour le traitement de troubles qui sont associés à un rôle de l'endothéline et exigent un blocage ET_{A} sélectif pour le traitement.

12. Composé selon l'une quelconque des revendications 1 à 4 pour utilisation en tant que médicament pour le traitement de troubles qui sont associés à un rôle de l'endothéline et exigent un blocage ET_{B} sélectif pour le traitement.

13. Utilisation d'un ou de plusieurs composés selon l'une quelconque des revendications 1 à 4, en tant que constituants actifs pour la production de compositions pharmaceutiques pour le traitement de troubles associés à un rôle de l'endothéline.

14. Utilisation selon la revendication 13, dans laquelle les troubles associés à un rôle de l'endothéline sont des troubles de la circulation.

15. Utilisation selon la revendication 13, dans laquelle les troubles associés à un rôle de l'endothéline sont des troubles inflammatoires.

16. Utilisation selon la revendication 13, dans laquelle les troubles associés à un rôle de l'endothéline sont des troubles de la prolifération.

17. Utilisation selon la revendication 13, dans laquelle les troubles associés à un rôle de l'endothéline sont l'hypertension, les maladies coronaires, l'insuffisance cardiaque, l'ischémie rénale et myocardique, la défaillance rénale, l'ischémie cérébrale, la démence, la migraine, l'hémorragie sous-arachnoïdienne, le syndrome de Raynaud, l'hypertension portale, l'hypertension pulmonaire, l'athérosclérose, la prévention de la resténose après angioplastie à ballon ou à stent, l'inflammation, la fibrose pulmonaire, les maladies des tissus connectifs, l'ulcère d'estomac et l'ulcère du duodénum, l'ulcère digital, le cancer, le mélanome, le cancer de la prostate, l'hypertrophie prostatique, la dysfonction érectile, la perte auditive, l'amaurose, la bronchite chronique, l'asthme, la septicémie gramme-négative, les chocs, la drépanocytose, la glomérulonéphrite, la colique rénale, le glaucome, la thérapie et la prophylaxie des complications diabétiques, les complications de la chirurgie vasculaire ou cardiaque ou après transplantation d'organes et les complications dues à la cyclosporine.

18. Utilisation selon la revendication 17, dans laquelle le ou les composés est ou sont destinés à une utilisation en combinaison à l'une ou à plusieurs substances utiles du point de vue thérapeutique.

19. Utilisation selon la revendication 18, dans laquelle la ou les susbtances utiles du point de vue thérapeutique est ou sont sélectionnées parmi le groupe constitué des agents α- et β-bloqueurs, des agents vasodilatateurs, des agents antagonistes du calcium, des agents inhibiteurs ACE, des agents activateurs du potassium, des agents antagonistes de récepteurs de l'angiotensine II, des agents diurétiques, des agents sympatholitiques et d'autres agents thérapeutiques qui servent à traiter la tension sanguine élevée ou tout autre trouble cardiaque.

20. Procédé en vue de la fabrication de compositions pharmaceutiques pour le traitement de troubles associés à un rôle de l'endothéline, contenant un ou plusieurs composés selon les revendications 1 à 4, en tant que constituants actifs, lequel procédé comprend le mélange d'un ou de plusieurs constituants actifs à des excipients acceptables du point de vue pharmaceutique d'une manière connue en soi.
